# EUROPÄISCHE PATENTANMELDUNG

(11) **EP 1 516 662 A1**
(43) Veröffentlichungstag der Anmeldung: **23.03.2005**
(21) Anmeldenummer: 04405426.0
(22) Anmeldetag: 07.07.2004
(51) Int. Cl.: B01F 17/00, B01F 3/08, A23L 1/035, B01F 17/14, C12N 5/00

(54) **Zubereitung bestehend aus mindestens zwei Nanoemulsionen**

(30) Priorität: 28.08.2003 CH 14662003
(71) Anmelder: Mibelle AG, 5033 Buchs (CH)
(72) Erfinder: Zülli, Fred, 5024 Küttigen (CH); Suter, Franz, 5312 Döttingen (CH); Liechti, Christina, 5004 Aarau (CH); Belser Gisi, Esther, 5033 Buchs (CH)
(74) Vertreter: Rottmann, Maximilian R.

(57) **Zusammenfassung**

Die stabilen Zusammensetzungen bestehen aus mindestens zwei verschiedenen, durch Lecithin stabilisierten Nanoemulsionen, von welchen jede ein flüssiges Lipid enthält, von welchen Lipiden mindestens zwei miteinander nicht kompatibel sind. Es werden Verfahren zu deren Herstellung beschrieben. Die Zusammensetzungen finden in der Kosmetik, in Zellkulturen und in Nahrungsergänzungsmitteln Verwendung.

## Beschreibung

Die Erfindung betrifft:
- die in den Ansprüchen 1 bis 13 umschriebenen Zubereitungen aus mindestens zwei Nanoemulsionen;
- die in den Ansprüchen 14 bis 16 umschriebenen Verfahren zur Herstellung solcher Zubereitungen; und
- die in den Ansprüchen 16 bis 18 umschriebenen Verwendungen solcher Zubereitungen.

Nanoemulsionen, auch Nanopartikel genannt, bestehen bekanntlich aus Öltröpfchen, die an ihrer Oberfläche mit einem amphoteren Emulgator besetzt sind, in wässrigen Systemen. Als Emulgator können Lecithin oder andere Emulgatoren, wie z.B. Poloxamere (internationales Freizeichen für Copolymere aus Polyethylenglykolen und Polypropylenglykolen) oder Natriumcholat, verwendet werden. Die Besetzung der Öltröpfchen mit dem amphoteren Emulgator an ihrer Oberfläche ist zweckmässig einlagig. Zweckmässigerweise enthalten die Nanoemulsionen pro Gewichtsteil Öl mehr als 0,4 Gewichtsteile, vorzugsweise 0,45 bis 1,0 Gewichteile, des amphoteren Emulgators. Der Durchmesser der Öltröpfchen beträgt üblicherweise zwischen 20 und 1000 nm.

Zweckmässigerweise weisen die Nanoemulsionen ein negatives Zeta-Potential auf, vorzugsweise zwischen -10 mV und -50 mV, insbesondere zwischen -30 mV und -40 mV. Für spezielle Anwendungen können aber auch Nanoemulsionen mit einem positiven Zeta-Potential von Vorteil sein. Solche kationischen Nanoemulsionen können beispielsweise durch Zugabe eines C8- bis C22-Alkylamids erhalten werden.

Nanoemulsionen sind beliebig mischbar mit Wasser, sehr stabil und können sogar autoklaviert werden.

Nanoemulsionen können hergestellt werden durch Vermischen von Lipiden, z.B. Triglyceriden, in wässeriger Phase mit Lecithin in einem Hochdruckhomogenisator (z.B. Microfluidizer®). Die Herstellung solcher Nanoemulsionen ist zum Beispiel beschrieben in EP-B1-0 406 162.

Nanoemulsionen werden z.B. in der Kosmetik eingesetzt für den Wirkstoffstransport in tiefere Hautschichten. In Zellkulturen werden Nanoemulsionen eingesetzt zur Ergänzung der wässrigen Medien mit lipophilen Stoffen (US-B1-6 265 180), damit z.B. die Produktion von Antikörpern verbessert wird. Des Weiteren werden Nanoemulsionen eingesetzt zur Bestimmung der Biokompatibilität/Toxizität von Lipiden im Zellkultur-Tests (US-B1-6 265 180). Ferner werden Nanoemulsionen auch verwendet in Nahrungsergänzungsmitteln zur Erhöhung der Bioverfügbarkeit von lipophilen Stoffen, wie z.B. Coenzym Q10 in wässrigen Produkten.

Für die Herstellung von Nanoemulsionen werden üblicherweise flüssige Öle oder ein Gemisch verschiedener lipophiler Stoffe oder Öle verwendet. Nun kann es vorkommen, dass die einzusetzenden lipophilen Substanzen nicht miteinander kompatibel sind und miteinander reagieren. In diesem Falle können sie somit nicht miteinander in Nanoemulsionen verarbeitet werden.

Im Speziellen ist es z.B. nicht möglich, Tocopherol und Coenzym Q10 miteinander in Nanoemulsionen zu verarbeiten. Durch Elektronentransfer-Prozesse reagieren die beiden lipophilen Stoffe miteinander, und die Lösung verfärbt sich braun. Herkömmliche Emulsionen (Cremen) können auch nicht hergestellt werden, da die inkompatiblen Stoffe in der Creme reagieren. Werden die zwei Stoffe einzeln in die Emulsionen verarbeitet und die Emulsionen danach vermischt, reagieren die inkompatiblen Lipide trotzdem miteinander, da sich die Öltröpfen mit der Zeit vermischen.

Für die Herstellung von Nanoemulsionen werden üblicherweise flüssige Öle verwendet. Diese Öle können auch aus mehreren Komponenten bestehen. Dabei können auch lipophile Stoffe in den Ölen gelöst werden, wobei das entsprechende Gemisch bei Raumtemperatur flüssig ist.

Beschrieben wurde auch die Herstellung von so genannten Solid-Lipid-Nanopartikeln (SLN). Diese Dispersionen enthalten bei Raumtemperatur feste Lipidpartikel, die mit einem Emulgator (z.B. Lecithin) in Wasser dispergiert sind. Diese Dispersionen werden hergestellt, indem das Lipid geschmolzen wird und dann bei hoher Temperatur zu einer Nanoemulsion verarbeitet wird. Beim Abkühlen entstehen dann wieder feste Lipidpartikel. Diese Partikel eignen sich als "Controlled-release"-Vehikel für schlecht wasserlösliche Wirkstoffe, die in den Lipidpartikeln gelöst werden und dann langsam in die Wasserphase diffundieren (US-B1-6 207 178). Nachteile dieser Solid-Lipid-Lipidpartikel ist, dass die lipophilen Substanzen als Feststoffe nur eine sehr schlechte Bioverfügbarkeit aufweisen, sowohl in der Kosmetik als auch in Zellkulturen und als Nahrungsergänzungsmittel. Ein weiterer Nachteil dieser SLN-Dispersionen ist es, dass die Lipide auf die Schmelztemperatur erhitzt und bei dieser Temperatur verarbeitet werden müssen. Dieses Verfahren ist technisch im grossen Massstab aufwendig und die Lipide und/oder die Wirkstoffe können dabei zerstört werden.

Ein interessanter Stoff mit vielen Anwendungsmöglichkeiten in der Kosmetik, in Zellkulturen und in Nahrungsergänzungsmitteln ist Coenzym Q10 (Ubiquinone). Dieses Lipid ist bei Raumtemperatur fest und schmilzt bei ca. 50 °C. US-B1-6 197 349 beschreibt die Herstellung einer Nanoemulsion mit Coenzym Q10 [CoQ10] in Form einer unterkühlten Schmelze. Im Gegensatz zu den SLP-Nanodispersionen bleibt in diesem Fall das geschmolzene CoQ10 in der Nanoemulsion bei Raumtemperatur flüssig. Nachteil dieser Zubereitung ist es, dass zur Herstellung der Nanoemulsion sowohl die Wasserphase als auch die Lipidphase auf 70 °C erwärmt werden müssen. Die hohen Temperaturen sind schädlich für das CoQ10 und für weitere Aktivstoffe, die eingeschlossen werden. Ein weiterer Nachteil ist es, dass eine Ölphase, welche nur aus CoQ10 besteht, sich nicht für die Herstellung von sehr kleinen Öltröpfchen und hohen Konzentrationen von Coenzym Q10 eignet. Die Beispiele in US-B1-6 197 349 beschreiben ausschliesslich Nanoemulsionen mit einer Partikelgrösse von mehr als 67 nm. Die Nanoemulsionen mit über 3 Gew.-% Coenzym Q10 sind sogar grösser als 100 nm. Die Bioverfügbarkeit von kleinen Partikeln ist jedoch viel besser; insbesondere wenn sich diese Partikel dieselbe Grösse wie Viren haben (6 bis 50 nm).

Aufgabe der Erfindung war es deshalb, eine Zubereitung zu entwickeln, welche nicht kompatiblen Lipide völlig getrennt in verschiedenen Öltröpfchen enthält, welche miteinander gemischt werden können, ohne dass die Öltröpfchen miteinander reagieren. Diese Zubereitung soll auch eine gute Stabilität und Bioverfügbarkeit aufweisen.

Diese Aufgabe wird durch die im Anspruch 1 umschriebenen Massnahmen gelöst. Dabei werden mindestens zwei Nanoemulsionen separat hergestellt, wobei die inkompatiblen Substanzen oder Substanzgemische getrennt voneinander durch Hochdruck-Homogenisation in die Nanoemulsionen eingearbeitet werden. Werden diese Nanoemulsionen später miteinander zur erfindungsgemässen Zubereitung gemischt, entsteht eine Mehrfach-Nanoemulsion, in der die inkompatibeln Substanzen durch verschiedenen Kompartimente voneinander getrennt sind und daher nicht mehr miteinander reagieren können.

Erstaunlicherweise sind diese Zubereitungen (Mehrfach-Nanoemulsionen) nun sehr stabil und können bis zu 3 Jahren im Kühlschrank gelagert werden, ohne dass die inkompatiblen Substanzen miteinander reagieren. Zur Anwendung in Zellkultur-Tests können sie entweder autoklaviert oder sterilfiltriert (0,1 µm) werden, wenn sie einen kleinen Tröpfchendurchmesser aufweisen. Auf diese Weise können nun verschiedene inkompatible Lipide in derselben Zubereitung angeboten und appliziert werden. Dies vereinfacht die Anwendung in der Kosmetik, in Nahrungsergänzungsmitteln oder für Zellkulturtests erheblich.

Es ist zudem möglich, auf die beschriebene Weise mit Teilchen, welche kleiner als 80 nm sind, transparente Gemische herzustellen. Transparente Nanoemulsionen erlauben die Formulierung von ästhetisch ansprechenden, transparenten kosmetischen Hydrogelen. Beim Arbeiten mit Zellkulturen erleichtern transparente Nanoemulsionen die visuelle Kontrolle. Zudem ist die Bioverfügbarkeit dieser sehr kleinen Öltröpfchen in der Grösse von 20 bis 80 nm auf der Haut, in Zellkulturen und bei Nahrungsergänzungsmitteln stark erhöht.

Falls nun einer der zu verarbeitenden lipophilen Stoffe bei Raumtemperatur fest ist, muss er zuerst in einer lipophilen Trägerkomponente gelöst werden.

Der Erfindung liegt daher weiter die Aufgabe zugrunde, eine gegenüber dem Stand der Technik verbesserte Darreichungsform für feste lipophile Stoffe zu finden; insbesondere für den lipophilen Wirkstoff Coenzym Q10.

Diese Aufgabe wird durch eine Nanoemulsion gelöst, welche als flüssige Ölphase oder Ölphasen eine bei Raumtemperatur übersättigte Lösung oder übersättigte Lösungen des lipophilen Stoffes oder der lipophilen Stoffe in einem geeigneten Öl enthält.

Die Herstellung dieser Nanoemulsionen wird durch die Verwendung von Ölen erreicht, die sich sehr gut eignen für die Herstellung von ultra-kleinen Öltröpfchen durch Hochdruck-Homogenisation. Leider weisen jedoch viele interessante Wirkstoffe wie z.B. das Coenzym Q10 bei Raumtemperatur in diesen Trägerölen, die sich zur Herstellung von Nanoemulsionen eignen, nur eine geringe Löslichkeit auf. Die Löslichkeit dieser Wirkstoffe kann häufig signifikant erhöht werden, wenn die Trägeröle erwärmt werden und/ oder zusätzlich noch mit Alkohol oder anderen Lösungsmitteln verdünnt werden. Kühlt man jedoch diese bei höheren Temperaturen entstanden Lösungen wieder auf Raumtemperatur ab, so kristallisieren diese Wirkstoffe allmählich wieder aus. Werden diese erwärmten Lösungen jedoch gleich zu einer Nanoemulsion verarbeitet, entstehen erstaunlicherweise sehr stabile übersättigte Lösungen. Diese übersättigen Lösungen bleiben mehrere Jahre in der Nanoemulsion stabil erhalten, auch wenn diese bei 4 °C gelagert werden. Zur Charakterisierung des Aggregatszustandes des Coenzym Q10 in Nanoemulsionen können die Präparate mit Hilfe der Dynamischen Differenz-Kalorimetrie (Differential Scanning Calorimetry DSC) untersucht werden. Liegt das Coenzym Q10 in den Nanoemulsionen in flüssiger Form vor, kann mit der DSC Messung kein Schmelzvorgang bestimmt werden. Die Rekristallisation der Lipide aus übersättigen Lösungen führt meistens auch zur Zerstörung der Nanoemulsion, wobei zuerst die Partikel grösser werden und dann schlussendlich die Nanoemulsion auseinander bricht und sich die Lipide abscheiden.

Die beschriebenen übersättigten Nanoemulsionen weisen gegenüber Nanoemulsionen, welche aus einer unterkühlten Schmelze bestehen, eine Reihe von Vorteilen auf:
1. Die Lipide müssen nicht über den Schmelzpunkt erhitzt werden.
2. Die Wasserphase muss nicht erhitzt werden.
3. Dadurch ist das Herstellungsverfahren viel einfacher und weniger kostspielig.
4. Durch die freie Wahl eines geeigneten Trägeröles können viel kleinere Partikel hergestellt werden (z.B. unter 67 nm), die eine grössere Bioverfügbarkeit aufweisen.
5. Durch die freie Wahl eines Trägeröles können höhere Konzentrationen des Lipids in die Nanoemulsion verarbeitet werden, ohne dass dadurch die Partikelgrösse stark ansteigt

Mögliche Zusammensetzungen solcher Mehrfach-Nanoemulsionen und Mehrfach-Nanoemulsionen mit übersättigten Lösungen werden in den nachfolgenden Beispielen erläutert. Die Bezeichnung der Inhaltsstoffe entspricht der INCI- Nomenklatur (International Cosmetics Ingredients), wie sie zur Kennzeichnung kosmetischer Wirk- und Hilfsstoffe bestimmt ist.

### Beispiele

### Beispiel 1: Transparente Doppel-Nanoemulsion mit Coenzym Q10 und alpha-Tocopherol

| Nanoemulsion 1 - Zusammensetzung | |
|---|---|
| Lecithin | 3,5 Gew.-% |
| Tocopheryl Acetate | 3 Gew.-% |
| Caprylic/Capric Triglyceride | 3 Gew.-% |
| Ubiquinone (Coenzyme Q10) | 1 Gew.-% |
| Diisopropyl Adipate | 1 Gew.-% |
| Alcohol | 12 Gew.-% |
| Glycerin | 20 Gew.-% |
| Aqua | 59 Gew.-% |

### Herstellung von 1 kg Nanoemulsion 1

10 g Coenzym Q10 (Ubiquinone) werden bei 40°C in 30 g Tocopheryl Acetate und 30 g Caprylic/Capric Triglyceride gelöst. 35 g Lecithin werden in 120 g Alcohol gelöst und unter Rühren zu einem Gemisch von 590 g Wasser und 200 g Glycerin zugegeben. Die beiden Phasen werden vereinigt und dann 5-mal bei 1200 bar (1,2·10⁸ Pa) mit einem Hochdruckhomogenisator von Microfluidics Corp (MT 110®) homogenisiert. Die Messung der Partikelgrösse mit Hilfe der Photonkorrelationsspektroskopie (Autosizer 3C®) ergibt eine mittlere Partikelgrösse (Zav) von 63,4 nm.

| Nanoemulsion 2 - Zusammensetzung | |
|---|---|
| Lecithin | 5 Gew.-% |
| Caprylic/Capric Triglyceride | 4 Gew.-% |
| Tocopherol | 1 Gew.-% |
| Alcohol | 15 Gew.-% |
| Glycerin | 20 Gew.-% |
| Aqua | 55 Gew.-% |

### Herstellung von 1 kg Nanoemulsion 2

Die Nanoemulsion 2 wird entsprechend zur Nanoemulsion 1 hergestellt. Die Partikelgrösse beträgt 41,6 nm.

Die beiden Nanoemulsionen 1 und 2 werden im Verhältnis 1:1 gemischt. Die so hergestellte transparente Doppel-Nanoemulsion mit Coenzyme Q10 (Ubiquinone) und Tocopherol zeigt nun während mindestens 15 Monaten bei 4 °C, Raumtemperatur und bei 37 °C keine Verfärbungen, und die anfängliche Partikelgrösse von 68,6 nm bleibt stabil. Die Doppel-Nanoemulsion weist somit eine ausgezeichnete Lagerstabilität auf.

### Beispiel 2: Transparente Doppel-Nanoemulsion mit einer übersättigten Coenzym-Q10-Lösung

| Nanoemulsion 3 - Zusammensetzung | |
|---|---|
| Lecithin | 3 Gew.-% |
| Vegetable Oil | 4 Gew.-% |
| Ubiquinone (Coenzym Q10) | 1 Gew.-% |
| Alcohol | 20 Gew.-% |
| . Glycerin | 20 Gew.-% |
| Aqua | 52 Gew.-% |

### Herstellung von 1 kg Nanoemulsion 3

30 g Lecithin werden in 100 g Alkohol gelöst und unter Rühren zu 520 g Wasser und 200 g Glycerin zugegeben. 10 g Coenzym Q10 (Ubichinone) [CoQ10] werden bei 40°C in 40 g Vegetable Oil und 100 g Alkohol (Ethanol) gelöst. Wird diese CoQ10-Lösung wieder auf Raumtemperatur (25 °C) abgekühlt, so kristallisiert das meiste CoQ10 nach einigen Stunden wieder aus der Lösung aus. Deshalb wird die 40 °C warme CoQ10-Lösung zur Lecithin/Alkohol/Glycerin/Wasser Mischung gegeben und dann 6-mal bei 1200 bar (1,2·10⁸ Pa) mit einem Hochdruckhomogenisator von Microfluidics Corp (MT 110®) homogenisiert, wobei sich der Alkohol gleichmässig in der Nanoemulsion verteilt. Dabei entsteht eine orange transparente Nanoemulsion. Die Messung der Partikelgrösse mit Hilfe der Photonkorrelationsspektroskopie (Autosizer 3C®) ergibt eine mittlere Partikelgrösse (Zav) von 45,0 nm. Die Nanoemulsionsproben können bei 4 °C, bei Raumtemperatur und bei 37°C für ein Jahr inkubiert werden, ohne dass sich die Partikelgrösse wesentlich ändert. Die Messung mit dem Differenz-Kalorimeter (Perkins Eimer) ergibt keinen Phasenübergang für das Coenzym Q10 in der Nanoemulsion. Dies bedeutet, dass das Coenzym Q10 in gelöster Form in der Nanoemulsion vorhanden ist. Da bei 4 °C und bei Raumtemperatur die Löslichkeitsgrenze von Coenzym Q10 in Vegetable Oil klar überschritten ist, handelt es sich um eine stabile Nanoemulsion einer übersättigten Lösung.

Die Nanoemulsionen 3 und 2 werden im Verhältnis 1:1 gemischt. Die so hergestellte transparente Doppel-Nanoemulsion mit einer übersättigten Coenzyme-Q10-(Ubiquinone)-Lösung und Tocopherol zeigt während mindestens 20 Monaten bei 4 °C, bei Raumtemperatur und bei 37 °C keine Verfärbungen, und die anfängliche Partikelgrösse von 65,0 nm bleibt stabil. Die Doppel-Nanoemulsion weist daher eine ausgezeichnete Lagerstabilität auf.

### Beispiel 3: Doppel-Nanoemulsion mit verschiedenen Vitaminen

| Nanoemulsion 4 - Zusammensetzung | |
|---|---|
| Lecithin | 5 Gew.-% |
| Tocopheryl Acetate | 2 Gew.-% |
| Caprylic/Capric Triglyceride | 2 Gew.-% |
| Ubiquinone (Coenzyme Q10) | 0,5 Gew.-% |
| Retinyl Palmitate | 0,5 Gew.-% |
| Alcohol | 15 Gew.-% |
| Glycerin | 20 Gew.-% |
| Aqua | 55 Gew.-% |

### Herstellung von 1 kg Nanoemulsion 4:

Die Nanoemulsion 4 wird entsprechend zur Nanoemulsion 1 hergestellt. Die Partikelgrösse beträgt 56,3 nm.

| Nanoemulsion 5 - Zusammensetzung | |
|---|---|
| Lecithin | 3 Gew.-% |
| Caprylic/Capric Triglyceride | 3 Gew.-% |
| Tocopheryl Acetate | 2,5 Gew.-% |
| Borago Officinalis Seed Oil | 1 Gew.-% |
| Tocopherol | 0,4 Gew.-% |
| Ascorbyl Tetraisopalmitate | 0,1 Gew.-% |
| Alcohol | 15 Gew.-% |
| Glycerin | 20 Gew.-% |
| Aqua | 55 Gew.-% |

### Herstellung von 1 kg Nanoemulsion 5

Die Nanoemulsion 5 wird entsprechend zur Nanoemulsion 1 hergestellt. Die Partikelgrösse beträgt 61,4 nm.

Die beiden Nanoemulsionen 4 und 5 werden im Verhältnis 1:1 gemischt. Die so hergestellte transparente Doppel-Nanoemulsion mit Coenzyme Q10 (Ubiquinone) und Tocopherol zeigt während mindestens 12 Monaten bei 4°C, bei Raumtemperatur und bei 37 °C keine Verfärbungen, und die anfängliche Partikelgrösse von 63,1 nm bleibt stabil. Die Doppel-Nanoemulsion weist somit eine ausgezeichnete Lagerstabilität auf.

### Beispiel 4: Transparente Doppel-Nanoemulsion mit einer übersättigten Coenzym-Q10-Lösung mit sehr kleinem Tröpfchendurchmesser

| Nanoemulsion 6 - Zusammensetzung | |
|---|---|
| Lecithin | 5 Gew.-% |
| Ubiquinone | 3 Gew.-% |
| Caprylic/Capric Triglyceride | 2 Gew.-% |
| Alcohol | 20 Gew.-% |
| Glycerin | 20 Gew.-% |
| Aqua | 50 Gew.-% |

### Herstellung von 1 kg Nanoemulsion 6:

Die Nanoemulsion wird entsprechend zur Nanoemulsion 3 hergestellt. Es entsteht eine übersättigte stabile Nanoemulsion mit Coenzym Q10. Die Partikelgrösse beträgt 35,9 nm.

| Nanoemulsion 7 - Zusammensetzung | |
|---|---|
| Lecithin | 5 Gew.-% |
| Vitamin E Acetate | 0,9 Gew.-% |
| Caprylic/Capric Triglyceride | 2 Gew.-% |
| Tocopherol | 0,1 Gew.-% |
| Alcohol | 20 Gew.-% |
| Glycerin | 20 Gew.-% |
| Aqua | 50 Gew.-% |

### Herstellung von 1 kg Nanoemulsion 7

Die Nanoemulsion wird entsprechend zur Nanoemulsion 1 hergestellt. Die Partikelgrösse beträgt 27,5 nm.

Die beiden Nanoemulsionen 6 und 7 werden im Verhältnis 1:2 gemischt. Die so hergestellte transparente Doppel-Nanoemulsion mit Coenzyme Q10 (Ubiquinone) und Tocopherol zeigt während mindestens 12 Monaten bei 4 °C, bei Raumtemperatur und bei 37 °C keine Verfärbungen und die anfängliche Partikelgrösse von 32,5 nm bleibt stabil. Die Doppel-Nanoemulsion weist somit eine ausgezeichnete Lagerstabilität auf.

### Beispiel 5: Transparente Doppel-Nanoemulsion mit einem hohen Gehalt an Coenzym Q10 als übersättigte Lösung

| Nanoemulsion 8 - Zusammensetzung | |
|---|---|
| Lecithin | 5 Gew.-% |
| Ubiquinone | 8 Gew.-% |
| Vegetable Oil | 4 Gew.-% |
| Alcohol | 20 Gew.-% |
| Glycerin | 20 Gew.-% |
| Aqua | 43 Gew.-% |

### Herstellung von 1 kg Nanoemulsion 8

50 g Lecithin werden in 100 g Alkohol gelöst und unter Rühren zu 430 g Wasser und 200 g Glycerin zugegeben. 80 g Coenzym Q10 (Ubichinone) [CoQ10] werden bei 45 °C in 40 g Vegetable Oil und 100 g Alkohol (Ethanol) gelöst. Wird diese CoQ10-Lösung wieder auf Raumtemperatur (25 °C) abgekühlt, so kristallisiert das meiste CoQ10 nach einigen Stunden wieder aus der Lösung aus. Deshalb wird die 40 Grad warme CoQ10-Lösung zur Lecithin/Alkohol/Glycerin/Wasser-Mischung gegeben und dann 6-mal bei 1200 bar (1,2·10⁸ Pa) mit einem Hochdruckhomogenisator von Microfluidics Corp (MT 110®) homogenisiert, wobei sich der Alkohol gleichmässig in der Nanoemulsion verteilt. Dabei entsteht eine orange transparente Nanoemulsion. Die Messung der Partikelgrösse mit Hilfe der Photonkorrelationsspektroskopie (Autosizer 3C®) ergibt eine mittlere Partikelgrösse (Zav) von 38,7 nm. Die Nanoemulsionsproben können bei 4°C, bei Raumtemperatur und bei 37 °C während eines Jahr inkubiert werden, ohne dass sich die Partikelgrösse wesentlich ändert. Die Messung mit dem Differenz-Kalorimeter (Perkins Elmer) ergibt keinen Phasenübergang für das Coenzym Q10 in der Nanoemulsion. Dies bedeutet, dass das Coenzym Q10 in gelöster Form in der Nanoemulsion vorhanden ist. Da bei 4°C, bei Raumtemperatur und bei 37 °C die Löslichkeitsgrenze von Coenzym Q10 in Vegetable Oil klar überschritten ist, handelt es sich um eine stabile Nanoemulsion einer übersättigten Lösung.

| Nanoemulsion 9 - Zusammensetzung | |
|---|---|
| Lecithin | 5 Gew.-% |
| Vegetable Oil | 4 Gew.-% |
| Tocopherol | 1 Gew.-% |
| Alcohol | 20 Gew.-% |
| Glycerin | 20 Gew.-% |
| Aqua | 50 Gew.-% |

### Herstellung von 1 kg Nanoemulsion 9

Die Nanoemulsion 9 wird entsprechend zur Nanoemulsion 1 hergestellt. Es entsteht eine transparente Nanoemulsion mit einer Partikelgrösse von 33,6 nm.

Die beiden Nanoemulsionen 8 und 9 werden im Verhältnis 3:1 gemischt. Die so hergestellte transparente Doppel-Nanoemulsion mit Coenzyme Q10 (Ubiquinone) und Tocopherol zeigt während mindestens 12 Monaten bei 4°C, bei Raumtemperatur und bei 37 °C keine Verfärbungen, und die anfängliche Partikelgrösse von 38,6 nm bleibt stabil. Die Doppel-Nanoemulsion weist somit eine ausgezeichnete Lagerstabilität auf.

## Patentansprüche

1. Zubereitung, bestehend aus mindestens zwei verschiedenen, durch Lecithin stabilisierten Nanoemulsionen, von welchen jede ein flüssiges Lipid enthält, von welchen Lipiden mindestens zwei miteinander nicht kompatibel sind.

2. Zubereitung nach Anspruch 1, **dadurch gekennzeichnet, dass** sie bei Raumtemperatur mindestens 6 Monate stabil ist, ohne dass die inkompatiblen Lipide miteinander reagieren.

3. Zubereitung nach Anspruch 2, **dadurch gekennzeichnet, dass** sie bei Raumtemperatur mindestens 2 Jahre stabil ist, ohne dass die inkompatiblen Lipide miteinander reagieren.

4. Zubereitung nach einem der Ansprüche 1 bis 3, **dadurch gekennzeichnet, dass** die Partikelgrösse der Lipidtröpfchen in allen Nanoemulsionen kleiner als 80 nm ist, so dass sie transparent erscheint.

5. Zubereitung nach einem der Anspruch 4, **dadurch gekennzeichnet, dass** die Partikelgrösse der Lipidtröpfchen in allen Nanoemulsionen kleiner als 45 nm ist.

6. Zubereitung nach einem oder mehreren Ansprüchen 1 bis 5, **dadurch gekennzeichnet, dass** die nicht kompatiblen Lipide Tocopherol und Coenzym Q10 sind.

7. Zubereitung nach Anspruch 6, **dadurch gekennzeichnet, dass** die Konzentration an Tocopherol 0,1 bis 20 Gew.-% und die Konzentration an Coenzym Q10 0,1 bis 20 Gew.-% beträgt.

8. Zubereitung nach einem oder mehreren Ansprüchen 1 bis 7, **dadurch gekennzeichnet, dass** mindestens eine der Nanoemulsionen als flüssige Ölphase eine bei Raumtemperatur übersättigte Lösung eines lipophilen Stoffes in Öl enthält.

9. Zubereitung nach Anspruch 8, **dadurch gekennzeichnet, dass** die übersättigte Nanoemulsion bei 4 °C mindestens 6 Monate stabil ist.

10. Zubereitung nach Anspruch 9, **dadurch gekennzeichnet, dass** die übersättigte Nanoemulsion bei 4 °C mindestens 3 Jahre stabil ist.

11. Zubereitung nach einem der Ansprüche 8 bis 10, **dadurch gekennzeichnet, dass** der lipophile Stoff in einer der übersättigten Nanoemulsionen Coenzym Q10 ist.

12. Zubereitung nach Anspruch 11, **dadurch gekennzeichnet, dass** die übersättigte Nanoemulsion 0,1 bis 20 Gew.-% Coenzym Q10 enthält.

13. Verfahren zur Herstellung einer Zubereitung nach einem der Ansprüche 1 bis 12 mit mindestens einem Lipid, welches bei Raumtemperatur in fester Form vorliegt, **dadurch gekennzeichnet, dass** man mindestens eines der miteinander nicht kompatiblen Lipide, welches in fester Form vorliegt, bei einer Temperatur, bei welcher es in Öl löslich ist, von anderen Lipiden getrennt in Öl löst, die Lösungen aller Lipide durch Hochdruck-Homogenisation in je eine Nanoemulsion einarbeitet und diese Nanoemulsionen anschliessend auf Raumtemperatur abkühlt, wobei eine stabile übersättigte Lösung des festen Lipids entsteht, und dass man die einzelnen Lösungen zu der Zubereitung vereinigt.

14. Verfahren zur Herstellung einer Zubereitung nach einem der Ansprüche 1 bis 12 mit mindestens einem Lipid, welches bei Raumtemperatur in fester Form vorliegt, **dadurch gekennzeichnet, dass** man mindestens eines der miteinander nicht kompatiblen Lipide, welches in fester Form vorliegt, bei einer Temperatur, bei welcher es in einer Mischung aus Öl und einem organischen Lösungsmittel löslich ist, von anderen Lipiden getrennt in einer solchen Mischung löst, die Lösungen aller Lipide durch Hochdruck-Homogenisation in je eine Nanoemulsion einarbeitet und diese Nanoemulsionen anschliessend auf Raumtemperatur abkühlt, wobei eine stabile übersättigte Lösung des festen Lipids entsteht, und dass man die einzelnen Lösungen zu der Zubereitung vereinigt.

15. Verfahren nach Anspruch 14, **dadurch gekennzeichnet, dass** man als organisches Lösungsmittel Ethanol einsetzt.

16. Verwendung einer Zubereitung nach einem oder mehreren der Ansprüche 1 bis 13 in kosmetischen Präparaten.

17. Verwendung einer Zubereitung nach einem oder mehreren der Ansprüche 1 bis 13 in Zellkulturen.

18. Verwendung einer Zubereitung nach einem oder mehreren der Ansprüche 1 bis 13 in Nahrungsergänzungsmitteln.
